# EUROPÄISCHE PATENTSCHRIFT

(11) **EP 2 826 505 B1**
(45) Veröffentlichungstag und Bekanntmachung des Hinweises auf die Patenterteilung: **25.04.2018**
(21) Anmeldenummer: 14177209.5
(22) Anmeldetag: 16.07.2014
(51) Int. Cl.: A61M 1/16

(54) **Dialyseanlage mit Wärmerückgewinnung**
Dialysis system with heat recovery
Installation de dialyse dotée d'une récupération de chaleur

(30) Priorität: 18.07.2013 DE 102013107673
(43) Veröffentlichungstag der Anmeldung: 21.01.2015
(73) Patentinhaber: B. Braun Avitum AG, 34212 Melsungen (DE)
(72) Erfinder: Alvensleben, Oliver, 21029 Hamburg (DE)
(74) Vertreter: Winter, Brandl, Fürniss, Hübner, Röss, Kaiser, Polte - Partnerschaft mbB

(56) Entgegenhaltungen:
- WO-A1-2012/119799
- US-A- 4 351 731
- US-A- 4 715 959
- US-A- 4 804 474
- US-A1- 2013 126 430

## Beschreibung

Die vorliegende Erfindung betrifft eine Dialyseanlage mit Wärmerückgewinnung sowie zentralem Energiemanagement und insbesondere eine Dialyseanlage bestehend aus einer stationären Wasser-/Fluidversorgung sowie selektiv daran anschließbaren Dialysemaschinen vorzugsweise der mobilen Bauart gemäß dem Oberbegriff des Patentanspruchs 1.

### Hintergrund der Erfindung

Dialyseanlagen der einschlägigen Gattung bestehen allgemein aus einer stationären Wasserversorgung mit einer Anzahl von Wasser-Anschlussmöglichkeiten (Zapfstellen), an die selektiv vorzugsweise mobile Dialysemaschinen fluidtechnisch angeschlossen werden können. Derartige Dialysemaschinen sind u. a. mit internen Heizsystemen ausgerüstet, um das aus der stationären Wasserversorgung abgezapfte Wasser in der Dialysephase auf Körpertemperatur und in der Desinfektionsphase auf etwas unter Siedetemperatur (ca. 90°C) aufzuheizen. Die hierfür erforderliche Primärenergie wird den Dialysemaschinen in Form von elektrischem Strom bereitgestellt. Zusätzlich können die Dialysemaschinen jeweils mit integrierten Wärmetauschern versehen sein, um Wärmeenergie in der verbrauchten Dialysierflüssigkeit dem aus der stationären Wasserversorgung abgezapften Wasser oder der daraus frisch aufbereiteten Dialysierflüssigkeit zuzuführen und dadurch den Primärenergieverbrauch der einzelnen Dialysemaschinen zu senken.

Diese bekannten Konzepte beruhen folglich im Wesentlichen auf dem Prinzip der sogenannten Insellösung in Form von völlig unabhängig arbeitenden Dialysemaschinen, die jeweils für sich (individuell) die erforderlichen Funktionsstoffe wie Desinfektionsmittel oder die Dialysierflüssigkeit, etc. in jeweils geeignetem Zustand (Temperatur, Konzentration) sowie zu jeweils geeigneten Zeitpunkten zur Verfügung stellen.

### Stand der Technik

Wie vorstehend bereits angedeutet wurde, muss bei einer Dialysebehandlung die durch den Dialysator fließende Dialysierflüssigkeit auf eine voreingestellte Zieltemperatur - in der Regel ca. 37°C - erwärmt werden. In der Dialysemaschine wird hierbei die einfließende Dialysierflüssigkeit/Wasser zunächst durch den vorstehend genannten internen Wärmetauscher vorgewärmt und anschließend durch eine Heizung auf den Zielwert vorzugsweise elektrisch aufgeheizt. Jedoch ist die Ausrüstung jeder Dialysemaschine mit einem Wärmetauscher aufwändig und auch teuer. Je nach Anzahl von angeschlossenen Dialysemaschinen wird daher die gesamte Dialyseanlage zunehmend kostenintensiv. Ein Verzicht auf den internen Wärmetauscher würde indessen die Betriebskosten aufgrund eines steigenden Primärenergieverbrauchs übergebührlich erhöhen.

In der WO 2012/175210 A2 ist ein Energieversorgungskonzept vorgeschlagen worden, das prinzipiell die Bereitstellung preisgünstig erzeugter Primärenergie vorsieht, welche beispielsweise in einem Dialysezentrum verbraucht wird, das mit einer Dialyseanlage gemäß vorstehendem Aufbau ausgestattet ist. Demnach ist u. a. eine Fotovoltaikanlage zur Erzeugung von elektrischem Strom mit einer Wärmepumpe insbesondere für das Erwärmen von Behandlungsräumen gepaart, welche hierfür thermische Energie aus einer Ablaufleitung für verbrauchte Dialysierflüssigkeit aufnimmt und Nutzenergie in die Behandlungsräume überträgt. Durch die Wärmepumpe kann somit die in der verbrauchten Dialysierflüssigkeit enthaltene Energie recycelt werden. Indessen bleibt das eingangs beschriebene Prinzip der Insellösung bezüglich der einzelnen Dialysemaschinen unberührt. US4804474A, US2013/126430A1 sind relevanter Stand der Technik.

### Kurzbeschreibung der Erfindung

Die Erfindung wird durch die Merkmale des unabhängigen Anspruchs 1 definiert. Der vorliegenden Erfindung liegt angesichts dieses Stands der Technik die Aufgabe zugrunde, ein Energieversorgungskonzept für ein Dialysezentrum bereit zu stellen, mittels dem die laufenden Betriebskosten des Dialysezentrums insgesamt verringert werden können.

Diese Aufgabe wird durch eine Dialyseanlage mit den Merkmalen des Anspruchs 1 gelöst. Vorteilhafte Weiterbildungen der Erfindung sind Gegenstand der Unteransprüche.

Der vorliegenden Erfindung liegen die nachfolgenden energetischen Überlegungen zugrunde:
Die Verbesserung der Betriebseffizienz eines Dialysezentrums beschränkt sich nicht nur auf die Bereitstellung preisgünstig erzeugter Primärenergie und das Recycling bereits eingesetzter Energie mittels bekannter Energieumwandlungsanlagen sondern erstreckt sich auch auf deren vorteilhaften Einsatz. Dazu ist es erforderlich, die monetären Aufwendungen in einem Dialysezentrum für Beschaffung, Wartung und laufenden Betrieb zu analysieren und durch geeignetes Zentralisieren der eingesetzten Energien zu optimieren.

Es hat sich beispielsweise gezeigt, dass insbesondere bei Dialysemaschinen deren individuelle Ausrüstung mit Wärmetauschern zu deutlichen Kostensteigerungen insbesondere für Beschaffung und Wartung (einschließlich Desinfektion) führt. Obgleich parallel hierzu laufende Betriebskosten etwa für die Beheizung des Dialysezentrums durch alternative Energieumwandlungsanlagen gemäß dem genannten Stand der Technik reduziert werden können, ist die dadurch erzielbare Kostenersparnis nach Erkenntnissen der vorliegenden Erfindung geringer als die vorstehend erwähnte Kostensteigerung. Es ist daher aus betriebswirtschaftlichen Erkenntnisses gemäß der Erfindung heraus vorteilhaft, die recycelte Wärmeenergie so einzusetzen, dass ggf. auf individuelle Wärmetauscher verzichtet werden kann oder diese zumindest kleiner dimensionierbar sind.

Auch kann nach neuen Erkenntnissen der individuelle Betrieb der Dialysemaschinen dazu führen, dass Dialysemaschinen zufällig in gleichen Betriebsphasen, etwa in einer Desinfektionsphase mit hohem Energieverbrauch betrieben werden. Dadurch kommt es zu temporären Stromverbrauchsspitzen, die tariflich bedingt zu überhöhten Stromgebühren führen. Der Einsatz der ggf. recycelten Primärenergie sollte demnach so gestaltet und gesteuert werden, dass eine Koordination der bisher nur individuell betriebenen Dialysemaschinen ermöglicht wird, um solche Verbrauchsspitzen zu vermeiden, zumindest aber zu reduzieren.

Gemäß einem ersten Aspekt ist die Dialyseanlage in ihrem stationären Abschnitt mit einer (zentralen) Wasser-Aufbereitungseinrichtung vorzugsweise der Osmosebauart ausgerüstet, die mit frischem (Leitungs-) Wasser bzw. Speisewasser versorgt wird und an deren Ausgang eine Wasserzuführleitung für (nicht stationäre) Dialysemaschinen angeschlossen ist, die einen Abschnitt mit einer Anzahl von Zweiganschlüssen hat. An diese Zweiganschlüsse sind in selektiver Weise vorzugsweise mobile Dialysemaschinen fluidgekoppelt bzw. koppelbar. Diese entsprechen hinsichtlich ihres Aufbaus im Wesentlichen aus dem Stand der Technik bekannten Maschinen, können jedoch ggf. ohne internen Wärmetauscher zur Energierückgewinnung ausgebildet sein. Ferner hat die Dialyseanlage eine Ablaufleitung, über die verbrauchte Dialysierflüssigkeit aus aktuell fluidgekoppelten Dialysemaschinen gesammelt in einen Abfluss oder einen Sammeltank entsorgbar ist. Erfindungsgemäß ist ein Dialysemaschinen-externer (stationärer/zentraler) erster Wärmetauscher (WT1) vorgesehen, dessen eine Seite an die Wasserzuführleitung zwischen der Wasser-Aufbereitungseinrichtung und einem in Strömungsrichtung hierzu nächst gelegenen Zweiganschluss bzw. dem Leitungsabschnitt mit den Zweiganschlüssen angeschlossen ist und dessen andere Seite an die Ablaufleitung und/oder den Sammelbehälter für die gesammelte verbrauchte Dialysierflüssigkeit angeschlossen ist.

In anderen Worten ausgedrückt, wird erfindungsgemäß unter Ausnutzung der in der verbrauchten Dialysierflüssigkeit enthaltenen Wärmeenergie unter Verwendung einer zentralen Heizung, insbesondere des ersten Wärmetauschers, das Wasser (Permeat) im stationären Abschnitt/Bereich der Anlage erwärmt und über die Wasserzuführleitung, vorzugsweise Ringleitung, den Dialysemaschinen zur Verfügung gestellt. Hierbei besteht grundsätzlich die Schwierigkeit, dass bei einem Erwärmen des Speisewassers für die Osmose das Risiko einer Verkeimung in der Wasser-Aufbereitungseinrichtung ansteigt. Da ferner für Osmosemembrane in der Regel Höchsttemperaturgrenzen gelten, ist die Höhe der Erwärmung des Speisewassers beschränkt und die Dialysemaschinen müssten zwangsläufig elektrisch nachheizen. Dadurch würde die Nutzung der Energierückgewinnung eingeschränkt.

Indessen ist es erfindungsgemäß vorgesehen, das bereits aufbereitete Wasser (Permeat) stromab von der Aufbereitungseinrichtung vorzuwärmen, sodass eine Temperaturerhöhung der Osmosemembran vermieden wird.

Vorzugsweise bildet die Wasserzuführleitung eine Ringleitung, die stromab zu der Anzahl von Zweiganschlüssen in Form einer Rücklaufleitung zur Wasser-Aufbereitungseinrichtung zurückgeführt ist. Dadurch geht ungenutztes, bereits vorerwärmtes Wasser (Permeat) nicht verloren, sondern wird über die Wasseraufbereitungseinrichtung oder unmittelbar stromab von dieser erneut dem ersten Wärmetauscher zur weiteren Vorheizung zugeführt.

Zusätzlich kann es vorgesehen sein, dass eine weitere Temperaturübertragungseinrichtung angeordnet ist, bestehend aus einem Dialysemaschinen-externen zweiten Wärmetauscher, dessen eine Seite an die Wasserzuführleitung zwischen der Wasser-Aufbereitungseinrichtung und dem ersten Wärmetauscher angeschlossen ist und dessen andere Seite an die Rücklaufleitung zwischen der Wasser-Aufbereitungseinrichtung und einem in Strömungsrichtung hierzu entferntest gelegenen Zweiganschluss bzw. dem Leitungsabschnitt mit den Zweiganschlüssen angeschlossen ist. Alternativ oder zusätzlich hierzu kann eine Wärmepumpe vorgesehen sein, welche thermische Energie aus der Ablaufleitung und/oder der Rücklaufleitung aufnimmt und Nutzenergie auf die Wasserzuführleitung zwischen der Wasser-Aufbereitungseinrichtung und dem in Strömungsrichtung hierzu nächst gelegenen Zweiganschluss bzw. dem Leitungsabschnitt mit den Zweiganschlüssen überträgt.

Mittels des zweiten Wärmetauschers findet also eine Vorerwärmung unter Ausnutzung der Restenergie in dem ungenutzt durch die Ringleitung geführten Wasser statt, wodurch sich das zur Aufbereitungseinrichtung zurückgeführte Wasser abkühlt und somit ein Aufheizen der Aufbereitungseinrichtung (und der darin befindlichen Osmosemembran) vermieden wird. Die Wärmepumpe hat den Vorteil, dass auch die Restenergie in der verbrauchten Dialysierflüssigkeit noch recycelt wird, um in das Wasser der Wasserzuführleitung eingespeist zu werden. Dabei ist insbesondere die Wärmepumpe elektronisch steuerbar, um so die Temperatur des Wassers in der Wasserzuführleitung auf einen geeigneten/gewünschten Wert zu erhöhen oder aber auch zu senken (in diesem Fall kann die Wärmepumpe in umgekehrter Weise als Kühleinrichtung betrieben werden).

Gemäß einem anderen Aspekt ist die erfindungsgemäße Dialyseanlage mit einer zentralen (stationären) elektronischen Steuereinheit ausgerüstet, an die zumindest ein Temperatursensor angeschlossen ist, der die Ist-Temperatur in der Wasserzuführleitung zumindest zwischen dem ersten Wärmetauscher (WT1) und dem die Zweiganschlüsse aufweisenden Leitungsabschnitt misst, wobei die Steuereinheit dementsprechend einen Wärmeübertragungsregler im Bereich des ersten Wärmetauschers und/oder die Wärmepumpe für das Erreichen einer Soll-Temperatur ansteuert, wodurch die übertragene oder übertragbare Wärmemenge durch den ersten Wärmetauscher und/oder durch die Wärmepumpe einstellbar ist. Dadurch kann zunächst prinzipiell einem Überhitzen des in der Ringleitung befindlichen Wasser vorgebeugt werden. Vorzugsweise ist die zentrale elektronische Steuereinheit zudem an die aktuell fluidgekoppelten Dialysemaschinen elektrisch angeschlossen oder anschließbar, um Informationen über deren aktuellen Betriebsphasen zu erhalten und in Abhängigkeit hiervon den Wärmeübertragungsregler und/oder die Wärmepumpe anzusteuern. Durch diese Rückkopplung wird ein Energiemanagement ermöglicht, wonach die Temperatur des in der Zuführleitung/Ringleitung befindlichen Wassers auf einen Wert eingestellt wird, der die notwendige Energie für ein individuelles Aufheizen des Wassers entsprechend der einzelnen Betriebsphasen der Dialysemaschinen auf ein Minimum reduziert. Vorteilhaft ist es hierfür, wenn die Soll-Temperatur in der Wasserzuführleitung/Ringleitung auf den kleinsten der von den aktuell angeschlossenen Dialysemaschinen geforderten Temperaturwerte eingestellt wird.

Gemäß einem anderen Aspekt kann es vorgesehen sein, dass die elektronische Steuereinheit in den Betriebsablauf der aktuell fluidgekoppelten Dialysemaschinen direkt/indirekt eingreift, um deren aktuelle Betriebsphasen so zu koordinieren, dass Betriebsphasen insbesondere mit hohem elektrischen Energieverbrauch zeitversetzt bei kleinstmöglicher zeitlicher Überlappung ausgeführt werden. Dadurch sind Stromverbrauchsspitzen vermeidbar/reduzierbar, sodass der Strom zu einem tariflich niedrigeren Preis eingekauft werden kann.

### Figurenbeschreibung

Die Erfindung wird nachstehend anhand eines bevorzugten Ausführungsbeispiels unter Bezugnahme auf die begleitende Figur näher erläutert. Diese zeigt das Fluid-Schaltbild einer Dialyseanlage sowie die elektronische Steuereinrichtung zur Überwachung und Steuerung der fluidmechanischen Elemente der Dialyseanlage sowie zur zentalen Koordination der aktuell angeschlossenen Dialysemaschinen bzw. deren aktuelle Betriebsphasen.

Erfindungsgemäß lässt sich die Dialyseanlage gemäß der vorliegenden Erfindung prinzipiell in einen stationären Bereich und einen instationären/mobilen Bereich untergliedern. Der mobile Bereich betrifft im Wesentlichen Dialysemaschinen, die getrennt voneinander sowie wahlweise an den stationären Bereich anschließbar sind oder in Betrieb genommen werden können, um jeweils eine Dialysebehandlung an einem Patienten auszuführen. Der stationäre Bereich beinhaltet u.a. ein Rohrleitungssystem mit darin integrierten strömungstechnischen Funktionselementen (Ventile, Drosseln, Filtern, etc) zur korrekten Versorgung der aktuell angeschlossenen/im Betriebszustand befindlichen Dialysemaschinen mit gereinigtem/vorbehandeltem Wasser sowie zur Entsorgung verbrauchter Fluide und eine zentrale Steuerung zur Bereitstellung des Wassers in einem gewünschten Zustand (gereinigt, temperiert, druckbeaufschlagt, etc) mittels hierfür geeigneter Funktionselemente (elektronische Steuereinheit, Temperatur-/Drucksensoren, Wärmetauscher, Wärmepumpen, Druckpumpen, etc.)

Die Dialysemaschinen entsprechen hinsichtlich ihres Aufbaus und ihrer Funktion im Wesentlichen aus dem Stand der Technik bekannten Maschinen, können jedoch bei der vorliegenden Erfindung wärmetauscherlos oder mit nur klein dimensioniertem internem Wärmetauscher ausgebildet sein. Außerdem weisen die Dialysemaschinen eine elektronische I/O-Schnittstelle auf, über die Daten/Informationen bezüglich der jeweils aktuellen Betriebszustände/Betriebsphasen vom Dialysemaschinen-internen Rechner (CPU) abgerufen und Steuersignale zur externen Beeinflussen der aktuellen Betriebszustände/Betriebsphasen eingegeben werden können. In sofern richtet sich die nachfolgende Beschreibung hauptsächlich auf den stationären Bereich der erfindungsgemäßen Dialyseanlage.

Dieser Bereich hat zunächst eine Speisewasser-Zuführleitung 1 mit integriertem Filter, ggf. Wasserenthärter und ggf. Drossel, über die Speisewasser aus einer Speisewasserquelle (örtliche Wasserversorgung, Brunnen, etc.) vorgereinigt und auf einen bestimmten Druck reduziert einer Wasser-Aufbereitungsanlage 2, vorzugsweise Osmosefilteranlage an deren Einlass zugeführt wird. In der Wasser-Aufbereitungsanlage 2 findet u. a. eine Wasserbehandlung statt, um das Speisewasser in ein dialyse-geeignetes Wasser aufzubereiten. Derartige WasserAufbereitungsanlagen sind hinlänglich aus dem Stand der Technik bekannt, sodass sich eine weitere Beschreibung an dieser Stelle erübrigt.

An den Auslass dieser Wasser-Aufbereitungsanlage 2 ist eine Zuführleitung 4 für dialyse-geeignetes Wasser angeschlossen, die in einen Leitungsabschnitt 6 mit einer Anzahl von Anschlussstellen 10 mündet. An diese Anschlussstellen (Zapfstellen) 10 sind wahlweise Dialysemaschinen 8 gemäß vorstehender Ausführung fluidtechnisch angeschlossen. Der Leitungsabschnitt 6 mit der Anzahl von Anschlussstellen 10 geht in Stromrichtung gesehen in eine Rückführleitung 12 über, die in die Wasser-Aufbereitungsanlage 2, insbesondere an deren Einlass zurückgeführt ist. Dadurch ergibt sich eine stationäre Ringleitung, in der permanent dialyse-geeignetes Wasser zirkuliert und immer wieder einem Reinigungsprozess in der Wasser-Aufbereitungsanlage 2 unterzogen wird. Alternativ hierzu ist es aber prinzipiell auch möglich, dass die Rückführleitung unmittelbar stromab zur Wasser-Aufbereitungsanlage in die Zuführleitung für dialyse-geeignetes Wasser (nachfolgend nur noch als Wasserzuführleitung bezeichnet) einmündet, wobei in diesem Fall gereinigtes Wasser aus der Aufbereitungsanlage nur noch in dem Umfang in die Ringleitung eingespeist wird, um aus den Anschlussstellen abgezapftes Volumen zu ersetzen. Schließlich kann in die Wasserzuführleitung und/oder die Rückführleitung eine Druckpumpe eingesetzt sein, um die Wasserzirkulation bei bestimmter Strömungsgeschwindigkeit in Gang zu halten.

Des Weiteren ist eine Sammelleitung 14 für verbrauchte Dialysierflüssigkeit vorzugsweise mit einer Anzahl von Anschlussstellen vorgesehen, an die die ausgewählten Dialysemaschinen 8 angeschlossen sind, um verbrauchte Dialysierflüssigkeit über die Sammelleitung 14 abzuführen und in einem Abfluss oder Behälter zu entsorgen. Dies Sammelleitung 14 wird daher nachstehend auch als Abwasserleitung oder Ablaufleitung bezeichnet.

In die Wasserzuführleitung 4 zwischen der Wasser-Aufbereitungsanlage 2 und dem Leitungsabschnitt 6 mit der Anzahl von Anschlussstellen 10 ist ein erster stationärer Wärmetauscher WT1 an dessen einer Seite WT1/1 zwischengeschaltet, dessen andere Seite WT1/2 in die Ablaufleitung 14 zwischengeschaltet ist. Auf diese Weise wird dem in der Ablaufleitung 14 abfließenden Fluid Wärme entzogen, dem in der Wasserzuführleitung 4 strömenden Wasser zugeführt und dieses dadurch erwärmt.

Vorzugsweise ist der erste stationäre Wärmetauscher WT1 bezüglich seiner Wärmeübertragungsleistung elektronisch/elektrisch regelbar. Zu diesem Zweck ist der erste stationäre Wärmetauscher WT1 bzw. dessen elektrische Regeleinrichtung RE an eine zentrale elektronische Steuereinheit (CPU) CT1 elektrisch angeschlossen. Des Weiteren sind eine Anzahl von Temperatursensoren T1-T4 an die elektronische Regeleinheit CT1 angeschlossen, welche in Strömungsrichtung beabstandet an der Wasserzuführleitung 4 angeordnet sind, derart, dass zumindest ein Temperatursensor stromauf und stromab zum ersten Wärmetauscher WT1 platziert ist. Vorzugsweise sind zwei Temperatursensoren T1, T4 stromab zum Wärmetauscher WT1 sowie in Stromrichtung beabstandet angeordnet.

Zwischen dem ersten stationären Wärmetauscher WT1 und der Wasser-Aufbereitungsanlage 2 ist ein zweiter stationärer Wärmetauscher WT2 angeordnet, dessen eine Seite WT2/1 in die Wasserzuführleitung 4 zwischengeschaltet und dessen andere Seite WT2/2 in die Rückführleitung 12 zwischengeschaltet ist. Dabei befindet sich einer der Temperaturfühler T2 an der Wasserzuführleitung 4 zwischen den beiden Wärmetauschern WT1, WT2 und ein weiterer Temperaturfühler T3 an der Wasserzuführleitung 4 zwischen dem zweiten stationären Wärmetauscher WT2 und der Wasser-Aufbereitungsanlage 2. Auf diese Weise erhält die elektronische Steuereinheit CT1 Informationen über den Temperaturverlauf längs der Wasserzuführleitung 4 ausgehend von der Aufbereitungsanlage 2 bis zum Leitungsabschnitt 6 mit den Zweiganschlussstellen 10. Ferner ist die zentrale elektronische Steuereinheit CT1 an die aktuell an die Ringleitung fluid-angeschlossenen Dialysemaschinen 8 an deren jeweiligen I/O-Schnittstellen elektrisch angeschlossen, um Informationen über deren aktuelle Betriebszustände/Betriebsphasen zu erhalten und regelnd in deren aktuelle Betriebszustände/Betriebsphasen nach bestimmten Kriterien gemäß nachfolgender Beschreibung ggf. einzugreifen.

Schließlich ist eine stationäre Wärmepumpe WP vorgesehen, welche thermische (Rest-) Energie aus der Ablaufleitung 14 und/oder der Rücklaufleitung 12 aufnimmt und Nutzenergie auf die Wasserzuführleitung 4 zwischen der Wasser-Aufbereitungseinrichtung 2 und dem die Zweiganschlüsse 10 aufweisenden Leitungsabschnitt 6 überträgt. Im Konkreten nimmt die Wärmepumpe WP noch verbleibende Wärmeenergie aus dem in der Ablaufleitung 14 stromab zum ersten Wärmetauscher WT1 abfliesenden Fluid auf und gibt Wärmeenergie in das in der Wasserzuführleitung 4 stromab zum zweiten Wärmetauscher WT2 fließenden Wassers vorzugsweise im Bereich des ersten Wärmetauschers WT1 ab.

Die Funktionsweise der erfindungsgemäßen Dialyseanlage mit dem vorstehenden Aufbau lässt sich wie Folgt beschreiben:
Grundsätzlich wird in der Dialyseanlage der vorliegenden Erfindung Speisewasser in der Wasser-Aufbereitungsanlage 2 gereinigt, sodass es eine für die nachfolgende Dialysebehandlung geeignete Qualität erlangt. Anschließend wird das aufbereitete Wasser in die stromabwärtige Ringleitung geführt, in der es von der Wasserzuführleitung 4 und dem die Zweiganschlüsse 10 aufweisenden Leitungsabschnitt 6 in die Rückführleitung 12 gelangt und von dort wieder zur Wasser-Aufbereitungsanlage 2 gemäß einem Wasserkreislauf zurückgeleitet wird.

Mit Durchströmen des zweiten Wärmetauschers WT2 unmittelbar stromab zur Wasser-Aufbereitungsanlage 2 erfährt das Wasser in der Wasserzuführleitung 4 zunächst eine Vorerwärmung, indem Wärmeenergie der Rückführleitung 12 entzogen und in die Wasserzuführleitung 4 übertragen wird. Dadurch kommt das zur Aufbereitungsanlage 2 rückgeführte Wasser kalt dort an, sodass eine Temperaturerhöhung beispielsweise einer Osmosemembran in der Aufbereitungsanlage 2 vermieden wird.

Anschließend durchströmt das Wasser in der Wasserzuführleitung 4 den ersten Wärmetauscher WT1, der nunmehr in geregelter Weise Wärmeenergie aus der die verbrauchte Dialysierflüssigkeit fördernden Ablaufleitung 14 in die Wasserzuführleitung 4 überträgt. Zusätzlich wird optional die Wärmepumpe WP betrieben, welche die vom ersten Wärmetauscher WT1 nicht genutzte Restenergie in der verbrauchten Dialysierflüssigkeit der Ablaufleitung 14 entzieht und der zum ersten Wärmetauscher WT1 stromabwärtigen Abschnitt der Wasserzuführleitung 4 zuführt. Alternativ ist es aber auch möglich, die Wärmepumpe WP umgekehrt zu betreiben, um das Wasser in der Ringleitung zu kühlen, beispielsweise im Fall eines Desinfektionsvorgangs in einer Dialysemaschine 8, worauf die stark erwärmte Dialysemaschine 8 möglichst schnell auf Behandlungstemperatur abgekühlt werden muss.

Über die Temperaturfühler T1-T4 stromauf zum zweiten Wärmetauscher WT2, zwischen dem ersten und zweiten Wärmetauscher sowie stromab zum zweiten Wärmetauscher WT2 lässt sich der Temperaturverlauf längs der Wasserzuführleitung 4 messen, wobei die Messergebnisse der zentralen Steuereinheit CT1 zugeführt werden. Diese erhält auch Informationen von den aktuell angeschlossenen Dialysemaschinen 8 insbesondere darüber, in welcher aktuellen Betriebsphase sich die einzelne Dialysemaschine 8 befindet und welche Temperatur das zugeführte Wasser dafür entsprechend haben muss.

Ausgehend hiervon bestimmt die zentrale Steuereinheit CT1 den jeweils niedrigsten geforderten Temperaturwert als Soll-Wert und reguliert die Wassertemperatur über den ersten Wärmetauscher WT1 und/oder die Wärmepumpe WP auf diesen, seitens aller aktuell angeschlossenen Dialysemaschinen 8 geforderten niedrigsten Wert. Durch die zentrale Erwärmung und Steuerung wird somit die effektive Vorbereitungszeit der einzelnen Dialysemaschinen 8 reduziert. Darüber hinaus hat die zentrale Steuereinheit CT1 gemäß der Erfindung noch weiterreichende Steuermöglichkeiten:
In Dialysezentren wird in der Regel im Schichtbetrieb gearbeitet. Der Zeitablauf ist also ähnlich und wiederholt sich kontinuierlich. Da die Dialysemaschinen 8 nach einer Dialysebehandlung grundsätzlich eine Desinfektionsphase durchlaufen müssen, erhöht sich der Gesamt-Strombedarf in Zentren in zyklischer Weise überproportional insbesondere dann, wenn mehrere Dialysemaschinen 8 gleichzeitig die Desinfektionsphase durchlaufen. Da Energieversorger neben dem reinen Stromverbrauch auch Gebühren in Abhängigkeit vom Spitzenstrombedarf berechnen, ist es sinnvoll, derartige Stromspitzen möglichst zu vermeiden oder zu minimieren.

Die Energie intensive Desinfektion einer Dialysemaschine läuft im Allgemeinen in den folgenden Schritten ab:
- Dialysierflüssigkeit ausspülen,
- Vorheizen,
- Desinfektionsmittel ansaugen,
- Desinfektionslösung aufheizen,
- heiße Lösung zirkulieren,
- heiße Lösung ausspülen,
- Dialysemaschine abkühlen.
Die Heizleistung je Maschine beträgt dabei zwischen 1500 bis 2000 W.

Die zentrale Steuereinheit CT1 erhält, wie vorstehend bereits angedeutet wurde, Informationen über die aktuellen Betriebsphasen der angeschlossenen Dialysemaschinen 8 und ist dafür angepasst, in die Dialysemaschinen-interne Steuerung einzugreifen, derart, dass durch zeitversetztes Starten der vorstehend genannten Aufheizphase (beispielsweise im Bereich von ca. 15 Minuten) der Spitzenstrombedarf gesenkt werden kann (um ca. 20% gegenüber gleichgeschalteten Dialysemaschinen). Basierend auf der vorstehenden Kommunikation zwischen zentraler Steuereinheit CT1 und den jeweils angeschlossenen Dialysemaschinen 8 ergibt sich bei entsprechenden zeitversetzten Aufheizphasen auch ein ebensolcher Zeitversatz für den jeweiligen Ausspülschritt, wodurch sich auch die max. Wassermenge reduziert, die ja beim Ausspülen genutzt wird.

In der dialysefreien Zeit bleibt die Rezirkulation des Wassers in der Ringleitung bestehen. Ähnlich wie bei dem Kühlschrankprinzip kann eine Reduzierung der Temperatur zu einem verringerten Keimwachstum führen. Durch die Einbeziehung der Wärmepumpe WP in die Dialyseanlage gemäß der Erfindung in Verbindung mit der zentralen Steuereinheit CT1 kann die Wassertemperatur in der Ringleitung bei nicht in Betrieb befindlichen Dialysemaschinen auf einen geeigneten niedrigen Wert abgesenkt werden. Des Weiteren kann, wie vorstehend bereits angedeutet wurde, die Wärmepumpe WP dazu genutzt werden, die angeschlossenen Dialysemaschinen 8 nach der Desinfektionsphase schnell und gezielt abzukühlen.

Durch die Kommunikation zwischen stationärer Wasserversorgung (einschließlich Wasser-Aufbereitungsanlage) und Dialysetechnologie wird eine Schnittstelle geschaffen, welche neben den vorstehend genannten Effekten noch zu weiteren Nutzenvorteilen führt:
- Bei der zentralen Desinfektion kann eine Inline-Heißreinigung stattfinden. Hierbei werden nicht nur die Ringleitung (ggf. inklusiver der Wasser-Aufbereitungsanlage) desinfiziert sondern auch die Dialysemaschinen eingebunden. Über die zentrale Steuereinheit kann eine optimale Einbeziehung der Dialysemaschinen in die Heißreinigung eingestellt werden. Dies geschieht durch eine zentrale Ansteuerung der jeweiligen Dialysemaschinen nach Ablauf der Aufheizphase der Wasserversorgung. Die Dialysemaschinen werden in diesem Fall dazu veranlasst, die Ansaugphase der zentralen Heißreinigung zeitversetzt zu starten, sodass die Temperatur in der Versorgungsleitung im Wesentlichen aufrecht erhalten werden kann. Die Synchronisation dieses Vorgangs erfolgt somit zentral durch die Steuereinheit im Gegensatz zu der aktuell gängigen Praxis, wonach jede Maschine einzeln programmiert werden muss.
- Die Einbindung der zentralen Steuereinheit in die Synchronisation/Koordination der Betriebsphasensteuerung hat eine erhöhte Sicherheit zur Folge. In der Vergangenheit sind zahlreiche Vor-/Unfälle bekannt geworden, wonach sich Schlauchverbindungen zwischen den Zweiganschlüssen und den Dialysemaschinen selbständig gelöst haben oder beschädigt wurden, was zu hohen Wasserschäden im Dialysezentrum führte. Mit Hilfe der zentralen Steuereinheit kann jedoch eine Kommunikation zwischen den einzelnen Dialysemaschinen und der stationären Wasserversorgung stattfinden. Die Wasserversorgung bzw. deren Steuerung erhält Daten, wie viele Dialysemaschinen beispielsweise in die Heißreinigung eingebunden sind und wie viel Wasser hierfür bereitgestellt werden muss. Stimmt das Verhältnis zwischen dem in die Ringleitung geschickten und dem in die Wasser-Aufbereitungsanlage zurückkommenden Wasser nicht, kann die zentrale Steuereinheit auf das Vorhandensein eines Lecks schließen und die Anlage z.B. automatisch abschalten. Kostenintensive Schäden sind somit vermeidbar.

Zusammenfassend wird eine Dialyseanlage offenbart mit einer Wasser-Aufbereitungseinrichtung vorzugsweise der Osmosebauart, an deren Ausgang eine Wasserzuführleitung angeschlossen ist, die eine Anzahl von Zweiganschlüssen hat, an die in selektiver Weise Dialysemaschinen fluidgekoppelt sind und mit einer Ablaufleitung, über die verbrauchte Dialysierflüssigkeit aus fluidgekoppelten Dialysemaschinen entsorgbar ist. Erfindungsgemäß ist wenigstens ein Dialysemaschinen-externer Wärmetauscher vorgesehen, dessen eine Seite an die Wasserzuführleitung unmittelbar stromauf zu den Zweiganschlüssen und dessen andere Seite an die Ablaufleitung angeschlossen ist.

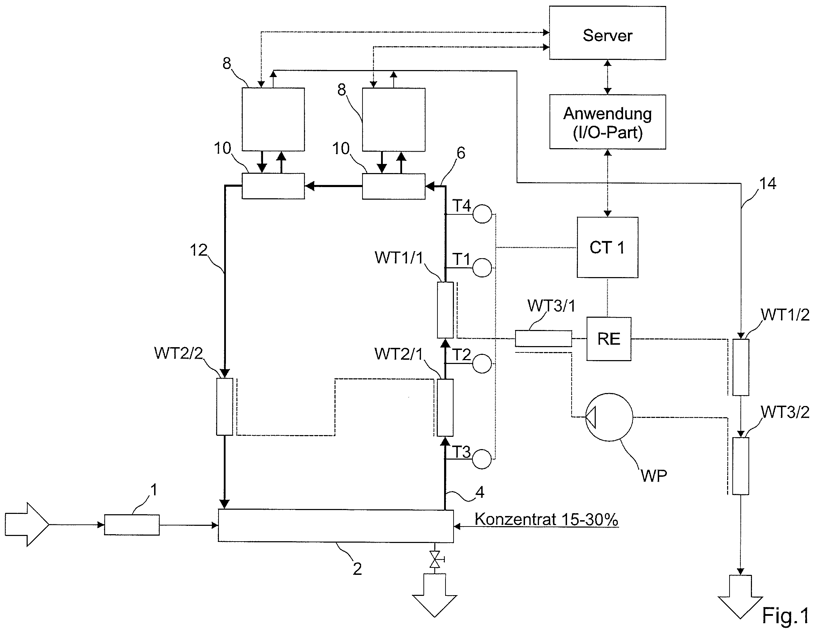

## Patentansprüche

1. Dialyseanlage mit
- einer Wasser-Aufbereitungseinrichtung (2) der Osmosebauart, an deren Ausgang eine Wasserzuführleitung (4) angeschlossen ist, die in zumindest einen Leitungsabschnitt (6) mit mehreren Zweiganschlüssen (10) mündet, an die in selektiver Weise mehrere Dialysemaschinen (8) fluidgekoppelt sind;
- einer Ablaufleitung (14), über die verbrauchte Dialysierflüssigkeit aus den fluidgekoppelten Dialysemaschinen (8) entsorgbar ist, und
- einem Dialysemaschinen-externen ersten Wärmetauscher (WT1), dessen eine Seite (WT1/1) an die Wasserzuführleitung (4) zwischen der Wasser-Aufbereitungseinrichtung (2) und dem die Zweiganschlüsse (10) aufweisenden Leitungsabschnitt (6) angeschlossen ist und dessen andere Seite (WT1/2) an die Ablaufleitung (14) angeschlossen ist.

2. Dialyseanlage nach Anspruch 1, bei der die Wasserzuführleitung (4) eine Ringleitung bildet und stromab zu dem die Anzahl von Zweiganschlüssen aufweisenden Leitungsabschnitt (6) in Form einer Rücklaufleitung (12) zur Wasser-Aufbereitungseinrichtung (2) zurückgeführt ist.

3. Dialyseanlage nach Anspruch 2, bei der eine weitere Temperaturübertragungseinrichtung bestehend aus
- einem Dialysemaschinen-externen zweiten Wärmetauscher (WT2), dessen eine Seite (WT2/1) an die Wasserzuführleitung (4) zwischen der Wasser-Aufbereitungseinrichtung (2) und dem ersten Wärmetauscher (WT1) angeschlossen ist und dessen andere Seite (WT2/2) an die Rücklaufleitung (12) zwischen der Wasser-Aufbereitungseinrichtung (2) und dem die Zweiganschlüsse aufweisenden Leitungsabschnitt (6) angeschlossen ist und/oder
- einer Wärmepumpe (WP), welche thermische Energie aus der Ablaufleitung (14) und/oder der Rücklaufleitung (12) aufnimmt und Nutzenergie auf die Wasserzuführleitung (4) zwischen der Wasser-Aufbereitungseinrichtung (2) und dem die Zweiganschlüsse aufweisenden Leitungsabschnitt (6) überträgt.

4. Dialyseanlage nach Anspruch 3, bei der im Fall einer Anordnung der Wärmepumpe (WP) diese stromab zum ersten Wärmetauscher (WT1) an die Ablaufleitung (14) angeschlossen ist und die Nutzenergie an die eine Seite (WT1/1) des ersten Wärmetauschers (WT1) abgibt.

5. Dialyseanlage nach Anspruch 3, bei der im Fall einer Anordnung der Wärmepumpe (WP) diese jeweils unmittelbar stromab zum ersten Wärmetauscher (WT1) an die Ablaufleitung (14) und die Wasserzuführleitung (4) angeschlossen ist.

6. Dialyseanlage nach einem der vorstehenden Ansprüche, bei der eine zentrale elektronische Steuereinheit (CT1), an die zumindest ein Temperatursensor (T1-T4) angeschlossen ist, der die Ist-Temperatur in der Wasserzuführleitung (4) zumindest zwischen dem ersten Wärmetauscher (WT1) und dem die Zweiganschlüsse aufweisenden Leitungsabschnitt (6) misst und die dementsprechend einen Wärmeübertragungsregler (RE) und/oder die Wärmepumpe (WP) für das Erreichen einer Soll-Temperatur ansteuert, wodurch die übertragene oder übertragbare Wärmemenge durch den ersten Wärmetauscher (WT1) und/oder durch die Wärmepumpe (WP) einstellbar ist.

7. Dialyseanlage nach Anspruch 6,bei der die zentrale elektronische Steuereinheit (CT1) an die aktuell fluidgekoppelten Dialysemaschinen (8) elektrisch angeschlossen ist, um Informationen über deren aktuellen Betriebsphasen zu erhalten und in Abhängigkeit hiervon den Wärmeübertragungsregler (RE) und/oder die Wärmepumpe (WP) so anzusteuern, dass sich die Soll-Temperatur in der Wasserzuführleitung (4) auf den kleinsten von einer der aktuell angeschlossenen Dialysemaschinen (8) geforderten Wert einstellt.

8. Dialyseanlage nach Anspruch 6,bei der die elektronische Steuereinheit (CT1) in den Betriebsablauf der aktuell fluidgekoppelten Dialysemaschinen (8) eingreift, um deren aktuelle Betriebsphasen so zu koordinieren, dass Betriebsphasen mit hohem elektrischen Energieverbrauch zeitversetzt bei kleinstmöglicher zeitlicher Überlappung ausgeführt werden.

## Claims

1. A dialysis system comprising
- a water treatment unit (2) of the osmosis type whose outlet has connected thereto a water supply line (4) that opens into at least one line section (6) provided with a plurality of branch connections (10) to which a plurality of dialyzers (8) are fluidly coupled in a selective manner;
- a drain line (14) through which the used dialysis fluid can be discharged from the fluid-coupled dialyzers (8), and
- a first heat exchanger (WT1) external to the dialyzer the one side (WT1/1) of which is connected to the water supply line (4) between the water treatment unit (2) and the line section (6) including the branch connections (10) and the other side (WT1/2) of which is connected to the drain line (14).

2. The dialysis system according to claim 1, in which the water supply line (4) defines a ring line, which, downstream of the line section (6) including the number of branch connections, is returned to the water treatment unit (2), said ring line being there configured as a feedback line (12).

3. The dialysis system according to claim 2, in which a further temperature transfer unit consisting of
- a second heat exchanger (WT2) disposed externally to the dialyzer the one side (WT2/1) of which is connected to the water supply line (4) between the water treatment unit (2) and the first heat exchanger (WT1), and the other side (WT2/2) of which is connected to the feedback line (12) between the water treatment unit (2) and the line section (6) including the branch connections
and/or
- a heat pump (WP) which absorbs thermal energy from the drain line (14) and/or the feedback line (12) and transfers useful energy to the water supply line (4) between the water treatment unit (2) and the line section (6) including the branch connections.

4. The dialysis system according to claim 3, in which, if the heat pump (WP) is arranged, said heat pump (WP) is connected to the drain line (14) downstream of the first heat exchanger (WT1) and transfers the useful energy to the one side (WT1/1) of the first heat exchanger (WT1).

5. The dialysis system according to claim 3, in which, if a heat pump (WP) is arranged, said heat pump (WP) is connected to the drain line (14) and the water supply line (4) immediately downstream of the first heat exchanger (WT1).

6. The dialysis system according to any one of the preceding claims, in which a central electronic control unit (CT1) to which at least one temperature sensor (T1-T4) is connected which measures the actual temperature in the water supply line (4) at least between the first heat exchanger (WT1) and the line section (6) including the branch connections and which controls in accordance therewith a heat transfer controller (RE) and/or the heat pump (WP) so as to accomplish a target temperature, thereby the transferred or transferable heat quantity being adjustable by the first heat exchanger (WT1) and/or by the heat pump (WP).

7. The dialysis system according to claim 6, in which the central electronic control unit (CT1) is electrically connected to the dialyzers (8) fluidly coupled at the time in question so as to obtain information on the prevailing operating phases thereof and which, in accordance with this information, controls the heat transfer controller (RE) and/or the heat pump (WP) such that the target temperature in the water supply line (4) adjusts to the smallest value required by one of the currently connected dialyzers (8).

8. The dialysis system according to claim 6, in which the electronic control unit (CT1) intervenes in the operating sequence of the dialyzers (8) fluidly coupled at the time in question, so as to coordinate their prevailing operating phases such that operating phases with high electric power consumption are performed with a time shift such that they temporally overlap one another as little as possible.

## Revendications

1. Installation de dialyse comprenant :
- un dispositif d'approvisionnement en eau (2) du type à osmose à la sortie duquel est raccordée une conduite d'alimentation en eau (4), qui débouche dans au moins une section de conduite (6) avec plusieurs jonctions de dérivation (10) auxquelles, de manière sélective, plusieurs machines de dialyse (8) sont couplées en communication fluidique ;
- une conduite de décharge (14) par laquelle le liquide de dialyse consommé peut être évacué des machines de dialyse (8) couplées en communication fluidique et
- un premier échangeur de chaleur (WT1) extérieur aux machines de dialyse, dont un côté (WT1-1) est raccordé à la conduite d'alimentation en eau (4) entre le dispositif d'approvisionnement en eau (2) et la section de conduite (6) présentant les jonctions de dérivation (10) et dont l'autre côté (WT1-2) est raccordé à la conduite de décharge (14).

2. Installation de dialyse selon la revendication 1, dans laquelle la conduite d'alimentation en eau (4) forme une conduite annulaire et est renvoyée sous la forme d'une conduite de retour (12) au dispositif d'approvisionnement en eau (2) en aval de la section de conduite (6) présentant le nombre de jonctions de dérivation.

3. Installation de dialyse selon la revendication 2, dans laquelle un autre dispositif de transmission de température constitué :
- d'un second échangeur de chaleur (WT2) extérieur aux machines de dialyse, dont un côté (WT2-1) est raccordé à la conduite d'alimentation en eau (4) entre le dispositif d'approvisionnement en eau (2) et le premier échangeur de chaleur (WT1) et dont l'autre côté (WT2-2) est raccordé à la conduite de retour (12) entre le dispositif d'approvisionnement en eau (2) et la section de conduite (6) présentant les jonctions de dérivation et/ou
- d'une pompe à chaleur (WP), qui reçoit de l'énergie thermique de la conduite de décharge (14) et/ou de la conduite de retour (12) et transfère de l'énergie recouvrable sur la conduite d'alimentation en eau (4) entre le dispositif d'approvisionnement en eau (2) et la section de conduite (6) présentant les jonctions de dérivation.

4. Installation de dialyse selon la revendication 3, dans laquelle, dans le cas d'un agencement de la pompe à chaleur (WP), celle-ci est raccordée à la conduite de décharge (14) en aval du premier échangeur de chaleur (WT1) et cède l'énergie recouvrable au premier côté (WT1-1) du premier échangeur de chaleur (WT1).

5. Installation de dialyse selon la revendication 3, dans laquelle, dans le cas d'un agencement de la pompe à chaleur (WP), celle-ci est raccordée respectivement directement en aval du premier échangeur de chaleur (WT1) à la conduite de décharge (14) et à la conduite d'alimentation en eau (4).

6. Installation de dialyse selon l'une quelconque des revendications précédentes, dans laquelle une unité de commande électronique centrale (CT1), à laquelle est connecté au moins un capteur de température (T1-T4), qui mesure la température réelle dans la conduite d'alimentation en eau (4) au moins entre le premier échangeur de chaleur (WT1) et la section de conduite (6) présentant les jonctions de dérivation et commande en conséquence un régulateur de transmission de chaleur (RE) et/ou la pompe à chaleur (WP) pour atteindre une température théorique, par laquelle la quantité de chaleur transmise ou transmissible peut être réglée par le premier échangeur de chaleur (WT1) et/ou la pompe à chaleur (WP).

7. Installation de dialyse selon la revendication 6, dans laquelle l'unité de commande électronique centrale (CT1) est connectée électriquement aux machines de dialyse (8) actuellement couplées en communication fluidique pour obtenir des informations sur leurs phases de fonctionnement actuelles et, en fonction de ces informations, commander le régulateur de transmission de chaleur (RE) et/ou la pompe à chaleur (WP) de sorte que la température théorique dans la conduite d'alimentation en eau (4) se règle sur la plus petite valeur fournie par l'une des machines de dialyse (8) actuellement raccordées.

8. Installation de dialyse selon la revendication 6, dans laquelle l'unité de commande électronique (CT1) s'engage dans le déroulement du fonctionnement des machines de dialyse (8) actuellement couplées en communication fluidique pour coordonner leurs phases de fonctionnement actuelles afin que les phases de fonctionnement avec une consommation d'énergie électrique élevée soient exécutées avec un décalage dans le temps en chevauchement temporel le plus petit possible.
